# EUROPEAN PATENT SPECIFICATION

(11) **EP 1 485 367 B1**
(45) Date of publication and mention of the grant of the patent: **01.08.2007**
(21) Application number: 03708344.1
(22) Date of filing: 11.03.2003
(51) Int. Cl.: C07D 307/87, A61K 31/343

(54) **PURIFIED CITALOPRAM SALTS**
GEREINIGTE CITALOPRAM SALZE
SELS DE CITALOPRAM PURIFIES

(30) Priority: 21.03.2002 GB 0206708
(43) Date of publication of application: 15.12.2004
(73) Proprietor: Cipla Ltd., Mumbai 400 008 (IN)
(72) Inventor: HAMIED, Yusuf, Khwaja, 2nd Floor, Windsor Villa, Mumbai 400026 (IN); KANKAN, Rajendra, N., A-3/5, NBD Society, Mumbai 400 084, Maharashtra (IN); RAO, Dharmaraj, R., Thane 400 601, Maharashtra (IN)
(74) Representative: Wain, Christopher Paul
(86) International application number: PCT/GB2003/001032
(87) International publication number: WO 2003/080589

(56) References cited:
- EP-A- 1 152 000
- WO-A-01/68627
- WO-A-01/80619
- WO-A-02/070501

## Description

This invention relates to purified pharmaceutical salts, more particularly to citalopram salts, and especially but not exclusively to purified citalopram hydrobromide and citalopram hydrochloride.

Citalopram is a well known antidepressant drug whose systematic name is 1-[3-(dimethylamino)propyl]-1-(4-fluorophenyl)-1,3-dihydro-5-isobenzofurancarbonitrile. It is a selective centrally acting serotonin (S-hydroxytryptamine; 5-HT) reuptake inhibitor. It is marketed as the hydrobromide or hydrochloride salt. One particular enantiomer of interest is S-citalopram and its salts.

Citalopram was first described in GB-A-1526331 and, subsequently, a number of different processes have been described for its preparation. The salts are prepared conventionally in crystalline form from the free base.

There are certain problems in practice in providing the hydrobromide and hydrochloride salts in an adequately pure form for pharmaceutical purposes. It is not uncommon for the crude citalopram base to contain various impurities arising from the synthetic route used for its preparation, and if crude base is converted directly to the hydrobromide or hydrochloride salt, some of these impurities can be very difficult to remove from the salts by conventional procedures. In view of this, it has been the practice to purify the base before forming the salt therefrom. The two main ways of achieving this have been by thin film distillation of the base, or by successive recrystallisations. Both these techniques have disadvantages.

We have now found another way of obtaining citalopram hydrobromide or hydrochloride in satisfactory pure form for use in pharmaceutical formulations. In particular, we have found that whilst it is very difficult to purify the hydrobromide or hydrochloride salts to remove various impurities, there are other salts whose purification does effect removal of these compounds. Accordingly, pure hydrobromide or hydrochloride can be made by first purifying another different salt, and then converting to the hydrobromide or hydrochloride.

In one aspect, the present invention provides a process for making purified citalopram hydrobromide or hydrochloride which comprises purifying another different citalopram salt and then converting the purified salt to the hydrobromide or hydrochloride.

The other different citalopram salt can be made in any suitable way such as by conventional techniques. The salt is one which can be purified to remove especially the impurities commonly present in crude citalopram base made by any of the various known syntheses, especially by cyanide exchange with a 5-halo atom, with or without catalysts. For this purpose, we prefer to use organic salts because inorganic salts of citalopram are more difficult to purify by crystallisation. Among the preferred organic salts are those of benzene sulphonic acid, p-toluene sulphonic acid, and methane sulphonic acid. Other salts which could be used are the acetate, ethanesulphonate, lactate, citrate, tartrate, bitartrate or maleate, or salts of fumaric, benzoic, ascorbic, succinic, salicyclic, bismethylenesalicyclic, propionic, gluconic, malic, malonic, mandelic, cinnamic, citraconic, stearic, palmitic, itaconic, glycolic and sulphamic acids.

The different salt can be purified in any way or ways as is convenient, but we prefer to purify it by crystallisation. The choice of solvent or solvent mixture will vary in dependence on the salt, but those skilled in the art will appreciate what solvents are best used. In general, we prefer to use one or more solvents selected from water, ethanol, methanol, acetone, ethyl acetate, toluene, hexane, heptane, methylethyl ketone and methyl isobutyl ketone.

The purified different salts are preferably converted directly to the desired hydrobromide or hydrochloride in known manner, but if desired purified free base can be formed from which the desired salt can subsequently be made. The purified hydrobromide and hydrochloride can be made in crystalline or amorphous form, as desired, or in the form of solutions or suspensions.

In accordance with the process of the present invention, it is possible to make the hydrobromide and hydrochloride salts, from crude citalopram base, the salts containing less than 0.1 % by weight of each of the well known 5-carboxamide, 5-chloro-, 5-bromo- and N-desmethyl impurities.

The invention further includes pharmaceutical compositions comprising purified citalopram hydrobromide or hydrochloride of the invention, and a pharmaceutically acceptable carrier. These compositions may be in any suitable form, such as tablets or capsules, as will be clear to those skilled in the art. Preferred compositions of the present invention contain S-citalopram, especially the hydrobromide.

In order that the invention may be more fully understood, the following Examples are given by way of illustration only.

### Example 1:

Crude citalopram base 10 g is dissolved in 100 ml toluene. To this is added benzene sulphonic acid 4.9 g. The mixture is stirred for 2 hr at room temperature and the reaction mixture is filtered to obtain citalopram besylate salt (12 g). (Purity 97%, by HPLC.)

### Example 2:

Crude citalopram besylate 25 g is dissolved in a mixture of toluene 100 ml and methanol 25 ml at 70°C and the solution is cooled to ambient temperature and stirred for 4 hr whereby the besylate salt of citalopram crystallises in pure form. (Purity >99.3%.)

### Example 3:

Pure citalopram besylate is dissolved in isopropanol and a molar quantity of hydrogen bromide gas is bubbled therethrough. The mixture is stirred for 2 hr at 50°C and cooled to 5°C for 2 hr and filtered to give pure citalopram hydrobromide.

## Claims

1. A process for making purified citalopram hydrobromide or hydrochloride which comprises purifying another different citalopram salt and then either converting the purified salt directly to the hydrobromide or hydrochloride, or converting the purified salt to purified citalopram base which is then converted to the hydrobromide or hydrochloride.

2. A process according to claim 1, wherein said another different salt is an organic salt.

3. A process according to claim 2, wherein the organic salt is a salt of benzene sulphonic acid, p-toluene sulphonic acid, or methane sulphonic acid.

4. A process according to claim 1, 2 or 3, wherein said another different salt is purified at least partly by crystallisation.

5. A process according to claim 4, wherein said salt is purified by crystallisation from a solvent comprising water, ethanol, methanol, acetone, ethyl acetate, toluene, hexane, heptane, methylethyl ketone or methyl isobutyl ketone, or any mixture of two or more thereof.

6. A process according to any of claims 1 to 5, wherein said another different salt is formed directly from citalopram base.

7. A process according to claim 6 wherein the citalopram base is crude citalopram base.

8. A process according to any of claims 1 to 7, wherein the purified hydrobromide or hydrochloride is made in crystalline form.

9. A process according to any of claims 1 to 8, wherein the purified hydrobromide or hydrochloride is made in amorphous form.

10. A process according to any preceding claim, wherein the citalopram is S-citalopram.

## Patentansprüche

1. Verfahren zur Herstellung von gereinigtem Citalopramhydrobromid oder -hydrochlorid, das das Reinigen eines anderen unterschiedlichen Citalopramsalzes und dann entweder das Umwandeln des gereinigten Salzes direkt in das Hydrobromid oder Hydrochlorid oder Umwandeln des gereinigten Salzes in eine gereinigte Citaloprambase beinhaltet, die dann in das Hydrobromid oder Hydrochlorid umgewandelt wird.

2. Verfahren nach Anspruch 1, wobei das genannte andere ' unterschiedliche Salz ein organisches Salz ist.

3. Verfahren nach Anspruch 2, wobei das organische Salz ein Salz von Benzolsulfonsäure, p-Toluolsulfonsäure oder Methansulfonsäure ist.

4. Verfahren nach Anspruch 1, 2 oder 3, wobei das genannte andere unterschiedliche Salz wenigstens teilweise durch Kristallisation gereinigt wird.

5. Verfahren nach Anspruch 4, wobei das genannte Salz durch Kristallisation von einem Lösungsmittel gereinigt wird, das Wasser, Ethanol, Methanol, Aceton, Ethylacetat, Toluol, Hexan, Heptan, Methylethylketon oder Methylisobutylketon oder jedes beliebige Gemisch aus zwei oder mehr davon umfasst.

6. Verfahren nach einem der Ansprüche 1 bis 5, wobei das genannte andere unterschiedliche Salz direkt aus der Citaloprambase gebildet wird.

7. Verfahren nach Anspruch 6, wobei die Citaloprambase eine Citalopramrohbase ist.

8. Verfahren nach einem der Ansprüche 1 bis 7, wobei das gereinigte Hydrobromid oder Hydrochlorid in kristalliner Form hergestellt wird.

9. Verfahren nach einem der Ansprüche 1 bis 8, wobei das gereinigte Hydrobromid oder Hydrochlorid in amorpher Form hergestellt wird.

10. Verfahren nach einem der vorherigen Ansprüche, wobei das Citalopram S-Citalopram ist.

## Revendications

1. Procédé de fabrication d'hydrobromure ou d'hydrochlorure de citaloprame purifié qui comprend la purification d'un autre sel de citaloprame différent puis soit la conversion directe du sel purifié en hydrobromure ou en hydrochlorure, soit la conversion du sel purifié en base de citaloprame purifiée qui est alors convertie en hydrobromure ou en hydrochlorure.

2. Procédé selon la revendication 1, dans lequel un autre sel différent est un sel organique.

3. Procédé selon la revendication 2, dans lequel le sel organique est un sel d'acide sulfonique de benzène, d'acide sulfonique de p-toluène, ou d'acide sulfonique de méthane.

4. Procédé selon la revendication 1, 2 ou 3, dans lequel ledit autre sel différent est purifié au moins en partie par cristallisation.

5. Procédé selon la revendication 4, dans lequel ledit sel est purifié par cristallisation à partir d'un solvant pouvant être l'eau, l'éthanol, le méthanol, l'acétone, l'acétate d'éthyle, le toluène, l'hexane, l'heptane, le méthyléthylcétone ou le méthylisobutylcétone, ou tout mélange de deux ou plus de ces solvants.

6. Procédé selon l'une quelconque des revendications 1 à 5, dans lequel l'autre sel différent est formé directement à partir de la base de citaloprame.

7. Procédé selon la revendication 6, dans lequel le citaloprame est une base de citaloprame brut.

8. Procédé selon l'une quelconque des revendications 1 à 7, dans lequel l'hydrobromure ou l'hydrochlorure purifié est élaboré sous forme cristalline.

9. Procédé selon l'une quelconque des revendications 1 à 8, dans lequel l'hydrobromure ou l'hydrochorure purifié est élaboré sous forme amorphe.

10. Procédé selon l'une quelconque des revendications 1 à 9 dans lequel le citaloprame est le S-citaloprame.
